(19) Europäisches Patentamt
European Patent Office
Office européen des brevets

(11) **EP 4 328 214 A1**

(12) **EUROPEAN PATENT APPLICATION**
published in accordance with Art. 153(4) EPC

(43) Date of publication:
**28.02.2024 Bulletin 2024/09**

(21) Application number: **22791722.6**

(22) Date of filing: **19.04.2022**

(51) International Patent Classification (IPC):
*C07C 29/74* (2006.01)     *C07C 31/125* (2006.01)
*A62D 3/176* (2007.01)     *A62D 101/24* (2007.01)
*B01J 19/12* (2006.01)     *B01J 19/24* (2006.01)

(52) Cooperative Patent Classification (CPC):
**A62D 3/176; B01J 19/12; B01J 19/24; C07C 29/74; C07C 31/125;** A62D 2101/24

(86) International application number:
**PCT/JP2022/018147**

(87) International publication number:
**WO 2022/224951 (27.10.2022 Gazette 2022/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.04.2021 JP 2021072426**

(71) Applicant: **Tokuyama Corporation**
**Shunan-shi, Yamaguchi 745-8648 (JP)**

(72) Inventors:
• **ABIKO Daisuke**
**Shunan-shi, Yamaguchi 745-8648 (JP)**
• **MIURA Kimihiro**
**Shunan-shi, Yamaguchi 745-8648 (JP)**
• **ICHINOSE Wataru**
**Shunan-shi, Yamaguchi 745-8648 (JP)**
• **ISOMURA Takenori**
**Shunan-shi, Yamaguchi 745-8648 (JP)**

(74) Representative: **Hoffmann Eitle**
**Patent- und Rechtsanwälte PartmbB**
**Arabellastraße 30**
**81925 München (DE)**

(54) **ULTRAVIOLET RAY IRRADIATION DEVICE, AND METHOD FOR PURIFYING ORGANIC COMPOUND**

(57)     Provided is an ultraviolet ray irradiation device, in which a flow tube through which a substance to be irradiated with ultraviolet ray flows is wound in a spiral manner around a bar-shaped ultraviolet ray source. Also provided is a method for purifying an organic compound that contains an organic lead compound as an impurity, the method comprising: a step for irradiating the organic compound with ultraviolet ray using the ultraviolet ray irradiation device; and a step for removing a lead component that is generated as the result of the irradiation of the organic lead compound with the ultraviolet ray from the organic compound.

FIG. 1

EP 4 328 214 A1

**Description**

TECHNICAL FIELD

The present invention relates to an ultraviolet irradiation device and a method for purifying an organic compound.

BACKGROUND ART

Strict control is required for the content of lead in organic compounds, such as those used in electronic materials and medical materials.

When synthesizing an organic compound, for example, an organomagnesium halide (Grignard reagent) is used. An organomagnesium halide is produced by reacting an organic halide with magnesium, and a trace amount of lead is present in magnesium as an impurity. Thus, organic compounds synthesized using an organomagnesium halide contain an organic lead compound as an impurity.

A method for purifying an organic compound containing an organic lead compound as an impurity is known. In this method, a solution of a resist monomer is washed using an aqueous solution of chlorine, bromine, or iodine (e.g., see Patent Document 1).

Patent Document 1: Japanese Unexamined Patent Application, Publication No.2003-128630

DISCLOSURE OF THE INVENTION

Problems to be Solved by the Invention

However, excess chlorine, bromine, or iodine needs to be removed from the washed solution of the resist monomer. Thus, a more efficient method for purifying an organic compound is desired.

Thus, the present applicant has proposed a method for purifying an organic compound containing an organic lead compound as an impurity by irradiating the organic compound with ultraviolet ray, but more efficient purification of an organic compound is desired.

An object of the present invention is to provide an ultraviolet irradiation device capable of efficiently purifying an organic compound containing an organic lead compound as an impurity, and a method for purifying an organic compound.

Means for Solving the Problems

According to an aspect of the present invention, an ultraviolet irradiation device includes a flow tube through which a substance to be irradiated with ultraviolet ray flows and the flow tube is wound in a spiral manner around a bar-shaped ultraviolet ray source.

The ultraviolet irradiation device described above can be used for purifying an organic compound containing an organic lead compound as an impurity.

Another aspect of the present invention is a method for purifying an organic compound containing an organic lead compound as an impurity. The method includes a step of irradiating the organic compound with ultraviolet ray using the ultraviolet irradiation device described above, and a step of removing a lead component that is generated as the result of the irradiation of the organic lead compound with the ultraviolet ray from the organic compound.

Effects of the Invention

According to the present invention, there can be provided the ultraviolet irradiation device capable of efficiently purifying an organic compound containing an organic lead compound as an impurity and the method for purifying an organic compound.

BRIEF DESCRIPTION OF THE DRAWINGS

FIG. 1 is a diagram illustrating an example of an ultraviolet irradiation device according to the present embodiment.
FIG. 2 is a diagram illustrating another example of a flow tube used in the ultraviolet irradiation device of FIG. 1.

PREFERRED MODE FOR CARRYING OUT THE INVENTION

Hereinafter, embodiments of the present invention will be described with reference to the drawings.

[Ultraviolet Irradiation Device]

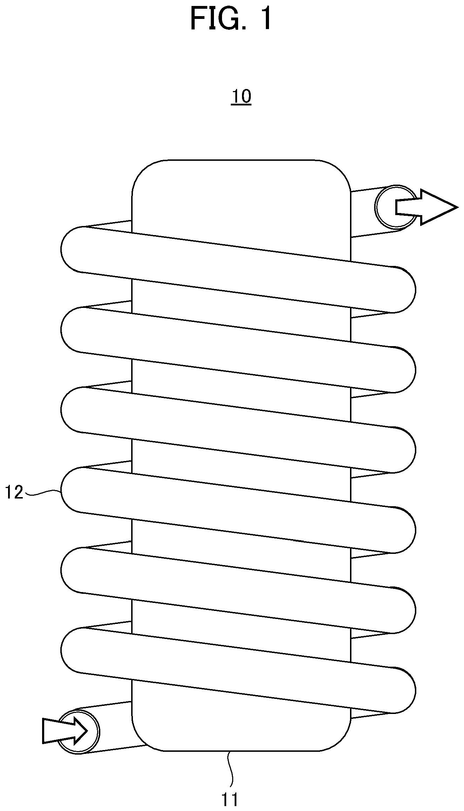

An example of an ultraviolet irradiation device according to the present embodiment is illustrated in FIG. 1.

In an ultraviolet irradiation device 10, a flow tube 12 through which a substance to be irradiated with ultraviolet ray flows is wound in a spiral manner around a straight and tubular ultraviolet ray source 11 at predetermined intervals. The ultraviolet ray is easily attenuated. Thus, the substance to be irradiated with ultraviolet ray flowing through the flow tube 12, which is in the vicinity of a region where the ultraviolet ray enters, can be efficiently irradiated. As a result, the substance to be irradiated with ultraviolet ray can be efficiently reacted. In addition, the ultraviolet irradiation device 10 is a continuous type and thus is useful for industrial production.

The substance to be irradiated with ultraviolet ray is not particularly limited as long as it can react when irradiated with ultraviolet ray, and examples include organic metals, such as organic lead compounds, organotin compounds, organozinc compounds, and organomagnesium compounds.

The form of the substance to be irradiated with ultraviolet ray that flows through the flow tube 12 is not particularly limited, and examples include a liquid such as a solution and a dispersion liquid, and a gas such as an aerosol.

To cause the substance to be irradiated with ultraviolet ray to flow through the flow tube 12, for example, a tube pump, a diaphragm pump, or the like can be used.

In addition, the substance to be irradiated with ultraviolet ray can be flowed through the flow tube 12 multiple times, that is, the substance to be irradiated with ultraviolet ray can be circulated through the flow tube 12.

Examples of the ultraviolet ray source 11 include, but are not limited to, an ultraviolet fluorescent lamp, a mercury lamp, a deuterium lamp, an ultraviolet light-emitting diode (LED), and an ultraviolet laser.

The shape of the ultraviolet ray source 11 is not limited to a straight and tubular shape as long as it is bar-shaped, and can be, for example, a U-tubular shape.

The length of the ultraviolet ray source 11 is not particularly limited and is, for example, 50 mm or more and 1000 mm or less.

The wavelength of the ultraviolet ray emitted by the ultraviolet ray source 11 is not particularly limited as long as the substance to be irradiated with ultraviolet ray can react at that wavelength.

A single ultraviolet ray source 11 is used in the ultraviolet irradiation device 10, but a plurality of the ultraviolet ray sources 11 can be used in combination.

In addition, although the ultraviolet irradiation device 10 is naturally air-cooled, the ultraviolet irradiation device 10 can be forcibly air-cooled by installing a fan, for example, below the ultraviolet ray source 11.

The material constituting the flow tube 12 is not particularly limited as long as it is an ultraviolet-transmissive material, one example being quartz.

The wall thickness of the flow tube 12 is not particularly limited and is, for example, 0.5 mm or more and 5 mm or less.

The diameter of the flow tube 12 is not particularly limited and is, for example, 3 mm or more and 40 mm or less.

The total length of the flow tube 12 is not particularly limited and is, for example, 3 m or more and 30 m or less.

The spiral diameter of the flow tube 12 wound in a spiral manner is not particularly limited and is, for example, 30 mm or more and 500 mm or less.

The distance between the ultraviolet ray source 11 and the flow tube 12 is not particularly limited and is, for example, 0 mm or more and 20 mm or less.

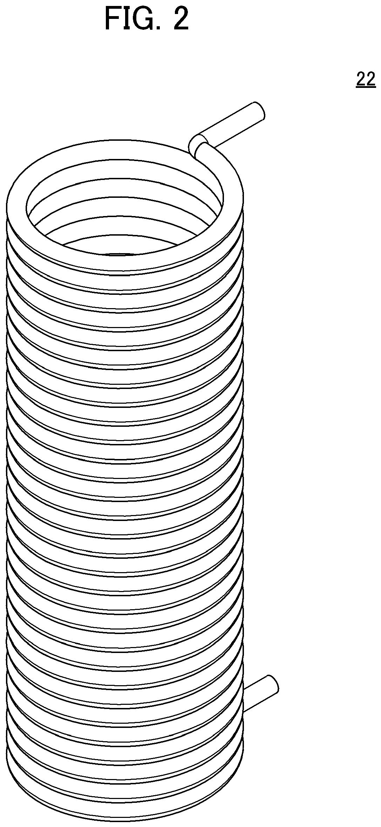

Another example of the flow tube used in the ultraviolet irradiation device of FIG. 1 is illustrated in FIG. 2.

A flow tube 22 is similar to the flow tube 12 except that the flow tube 22 is wound in a spiral manner without intervals. When the flow tube 22 is used, the substance to be irradiated with ultraviolet ray can be more efficiently irradiated with ultraviolet ray.

[Method for Purifying Organic Compound]

A method for purifying an organic compound according to the present embodiment is a method for purifying an organic compound containing an organic lead compound as an impurity.

The method for purifying an organic compound according to the present embodiment includes a step of irradiating an organic compound with ultraviolet ray using the ultraviolet irradiation device according to the present embodiment (e.g., the ultraviolet irradiation device 10) (hereinafter referred to as "ultraviolet irradiation step"), and a step of removing a lead component that is generated as the result of the irradiation of the organic lead compound with the ultraviolet ray from the organic compound (hereinafter referred to as "lead component removal step"). With this method, the organic compound can be efficiently irradiated with ultraviolet ray, and hence the organic compound can be efficiently purified.

(Organic Compound)

In the present embodiment, the organic compound can be synthesized using, for example, an organometallic compound.

Specific examples of the organic compound include tertiary alcohols and organosilicon compounds. Here, the tertiary alcohol can be obtained by, for example, reacting a Grignard reagent with a ketone compound. In addition, the organosilicon compound can be obtained by, for example, reacting a Grignard reagent with a silane compound.

Examples of the organometallic compound other than the Grignard reagent include organomagnesium halides, organocopper compounds, organozinc compounds, and organic rare earth compounds.

In the present embodiment, the organic compound can further contain an inorganic lead compound, such as lead chloride, lead, and/or the like as an impurity other than the organic lead compound.

Here, for example, lead chloride reacts with the Grignard reagent to form an organic lead compound. Accordingly, such a side reaction is considered to proceed during the synthesis of the organic compound or during the synthesis of the Grignard reagent to produce an organic compound containing an organic lead compound as an impurity.

In the present embodiment, the lead element content in the organic compound is not particularly limited and is, for example, 10 ppb or more and 1 ppm or less.

(Ultraviolet Irradiation Step)

Upon irradiation of the organic lead compound with ultraviolet ray, the carbon-lead bond of the organic lead compound is considered to be cleaved, that is, the organic lead compound is considered to be decomposed.

Examples of the lead component generated by decomposition of the organic lead compound include a lead ion and a lead salt.

The wavelength of the ultraviolet ray used to irradiate the organic compound is not particularly limited as long as the organic lead compound can be decomposed and is, for example, 210 nm or more and 350 nm or less. Using ultraviolet ray with a wavelength of 210 nm or more to irradiate the organic compound can inhibit decomposition of the organic compound. Using ultraviolet ray with a wavelength of 350 nm or less to irradiate the organic compound can facilitate decomposition of the organic lead compound. The wavelength of the ultraviolet ray used to irradiate the organic compound is preferably 220 nm or more and 320 nm or less, and more preferably 240 nm or more and 300 nm or less.

The illuminance of the ultraviolet ray used to irradiate the organic compound is not particularly limited as long as the organic lead compound can be decomposed and is, for example, 5 mW/cm$^2$ or more and 50 mW/cm$^2$ or less.

The flow rate when the organic compound flows through the flow tube is not particularly limited as long as the organic lead compound can be decomposed and is, for example, 5 mL/min or more and 200 mL/min or less.

The temperature of the organic compound when the organic compound flows through the flow tube is not particularly limited as long as the organic lead compound can be decomposed and is, for example, 0°C or higher and 30°C or lower.

The form of the organic compound that flows through the flow tube is preferably a solution in which the organic compound is dissolved in an organic solvent. With this configuration, the organic lead compound can be efficiently decomposed.

The organic solvent is not particularly limited as long as it does not have an absorption peak at the wavelength of the ultraviolet ray used to irradiate the organic compound. For example, a molar extinction coefficient ε of the organic solvent at the wavelength of the ultraviolet ray is 100 L·mol$^{-1}$·cm$^{-1}$ or less. When the molar extinction coefficient ε of the organic solvent at the wavelength of the ultraviolet ray is 100 L·mol$^{-1}$·cm$^{-1}$ or less, the organic compound can be efficiently decomposed. The molar extinction coefficient ε of the organic solvent at the wavelength of the ultraviolet ray is preferably 50 L·mol$^{-1}$·cm$^{-1}$ or less and more preferably 10 L·mol$^{-1}$·cm$^{-1}$ or less. The molar extinction coefficient ε of the organic solvent at the wavelength of the ultraviolet ray is usually 0.001 L·mol$^{-1}$·cm$^{-1}$.

Examples of organic solvents with high optical transparency at a wavelength of 210 nm or more and 220 nm or less include aliphatic hydrocarbon solvents, such as pentanes, hexanes, and heptanes; nitrile-based solvents, such as acetonitrile and propionitrile; and aliphatic lower alcohol solvents, such as methanol, ethanol, and propanol.

Examples of organic solvents with high optical transparency at a wavelength of 220 nm or more and 250 nm or less include ether-based solvents, such as diethyl ether, tetrahydrofuran, dioxane, and diisopropyl ether; and chlorine-based solvents, such as chloroform and dichloromethane.

Examples of organic solvents with high optical transparency at a wavelength of 250 nm or more and 350 nm or less include aromatic-based solvents, such as benzene and toluene; and ester-based solvents, such as ethyl acetate and propyl acetate.

The content of the organic compound in the solution is preferably 0.01 mass% or more and 10 mass% or less, and more preferably 0.01 mass% or more and 1 mass% or less. When the content of the organic compound in the solution is 0.01 mass% or more, the organic lead compound can be efficiently decomposed. When the content is 10 mass% or less, the lead component can be efficiently removed from the organic compound.

A photosensitizer can be added to the organic compound that flows through the flow tube. With this configuration, the organic lead compound can be efficiently decomposed.

Examples of the photosensitizer include benzophenone, anthracene, and camphorquinone.

The mass ratio of the photosensitizer to the organic compound is not particularly limited and is, for example, from 0.01 to 1.

When the organic compound has a polymerizable group, a polymerization inhibitor can be added.

Examples of the polymerizable group include a (meth)acrylic group, a vinyl group, and an epoxy group.

Examples of the polymerization inhibitor include dibutylhydroxytoluene and benzoquinone.

The mass ratio of the polymerization inhibitor to the organic compound is not particularly limited and is, for example, from 0.01 to 1.

(Lead Component Removal Step)

Examples of the lead component to be removed from the organic compound include a lead ion and a lead salt.

Here, when a lead ion, a lead salt, and/or the like is contained as an impurity of the organic compound together with the organic lead compound, the lead ion, lead salt, and/or the like contained as an impurity of the organic compound is also removed together with the lead component generated by irradiating the organic lead compound with the ultraviolet ray.

Examples of a method for removing the lead component include a method of washing the organic compound with water, a method of treating the organic compound with an adsorbent, and a method of filtering the organic compound. Two or more of these methods can be used in combination.

The form of the organic compound from which the lead component is removed is preferably a solution in which the organic compound is dissolved in an organic solvent. With this configuration, the lead component can be effectively removed from the organic compound.

The organic solvent is not particularly limited as long as it can dissolve the organic compound. However, if the organic compound is to be washed with water, a hydrophobic organic solvent needs to be used. When an organic solvent is used in the ultraviolet irradiation step, the organic solvent used in the ultraviolet irradiation step can be used as it is, or the organic solvent used in the ultraviolet irradiation step can be replaced with another organic solvent.

Upon washing the organic compound with water, the lead component moves to the aqueous layer, and thus the lead component can be removed from the organic compound by removing the aqueous layer. At this time, the operation of washing the organic compound with water can be performed multiple times.

The temperature of the water used for washing the organic compound is not particularly limited and is, for example, 0°C or higher and 30°C or lower.

At this time, an aqueous solution of an acid can be used instead of water. This can improve the solubility of the lead component.

Examples of the acid include nitric acid and hydrochloric acid.

The concentration of the acid in the aqueous solution of the acid is not particularly limited and is, for example, 0.001 mol/L or more and 1 mol/L or less.

Treating the organic compound with an adsorbent causes the lead component to be adsorbed on the adsorbent, and thus the lead component can be removed from the organic compound. At this time, the operation of treating the organic compound with the adsorbent can be performed multiple times.

The adsorbent is not particularly limited as long as it can adsorb the lead component, and examples include activated carbon, a cation exchange resin, a chelating resin, and a synthetic adsorbent.

The activated carbon can be in a granular form, a powdery form, or a fibrous form. Further, the activated carbon can be produced using either a raw material derived from a natural product, such as a coconut shell, or a synthetic resin and is preferably subjected to a heating and reduced-pressure drying treatment at a temperature of 150°C or higher and 250°C or lower as a pretreatment.

The temperature of the organic compound to be treated with activated carbon is not particularly limited and is, for example, 0°C or higher and 30°C or lower.

When the organic compound is treated with activated carbon, either batch treatment or column treatment can be used.

When batch treatment is used, 1 mass% or more and 15 mass% or less of activated carbon is added to the organic compound, and the mixture is stirred or shaken for 0.5 hours or more and 48 hours or less and then filtrated to remove the activated carbon.

When column treatment is used, a solution of the organic compound is passed through a column packed with activated carbon at a space velocity of 1 h$^{-1}$ or more and 50 h$^{-1}$ or less.

Examples of the synthetic adsorbent include a polystyrene-type synthetic adsorbent and a polymethacrylic acid-type synthetic adsorbent.

Specific examples of the polystyrene-type synthetic adsorbent include styrene-divinylbenzene copolymers and ethylstyrene-divinylbenzene copolymers.

Specific examples of the polymethacrylic acid-type synthetic adsorbent include methyl methacrylate-ethylene glycol dimethacrylate copolymers.

In the polystyrene-type synthetic adsorbent, the benzene ring of styrene is optionally replaced by a halogen atom, such as a bromine atom.

The cation exchange resin is not particularly limited as long as it can exchange a lead ion, and examples include a strongly acidic cation exchange resin, a weakly acidic cation exchange resin, a gel-type cation exchange resin, and a porous-type cation exchange resin.

The chelating resin is not particularly limited as long as it can capture a lead ion, and examples include an iminodiacetic acid-type chelating resin, a nitrilotriacetic acid-type chelating resin, an ethylenediaminetetraacetic acid-type chelating resin, a diethylenetriaminepentaacetic acid-type chelating resin, and a triethylenetetraminehexaacetic acid-type chelating resin.

When a synthetic adsorbent, a cation exchange resin, and a chelating resin are used, it is preferable to perform a known pretreatment and then replace with an organic solvent for dissolving the organic compound.

When the organic compound is treated with a synthetic adsorbent, a cation exchange resin, or a chelating resin, either batch treatment or column treatment can be used in the same manner as in treating the organic compound with activated carbon.

When the lead component is precipitated, the lead component can be removed from the organic compound by filtering the organic compound. Specifically, the organic compound is filtered using a filter, filter paper, or the like, and then the filtrate is collected.

(Application)

Using the method for purifying an organic compound according to the present embodiment can significantly decrease the lead element content in an organic compound containing an organic lead compound as an impurity and can also decrease the lead element content to a sub-ppb level on a mass basis. Thus, an organic compound produced by the method for purifying an organic compound according to the present embodiment can be applied to electronic materials, medical materials, and the like.

For example, a carbosilane compound containing an organic lead compound as an impurity is purified using the method for purifying an organic compound according to the present embodiment, and then the carbosilane compound is polycondensed by a known method. This can produce a polycarbosilane compound with a highly decreased lead element content.

Subjecting an organic compound produced by the method for purifying an organic compound according to the present embodiment to a known purification operation, such as recrystallization or column chromatography, can further improve the chemical purity.

EXAMPLES

Hereinafter, examples of the present invention will be described, but the present invention is not limited to the examples.

[Synthesis Example 1]

To a dried 10-L glass three-necked flask, 5 L of tetrahydrofuran (water content 10 ppm) and 80 g of fine powder magnesium were added, and then a solution in which 340 g of 1-bromopropane was dissolved in 500 mL of tetrahydrofuran (water content 10 ppm) was added dropwise using a dropping tube with agitation. The reaction solution generated heat along with the dropwise addition of the solution, and thus the solution was added dropwise over 2 hours while the flask was cooled in a water bath and the dropwise addition rate was adjusted. Thus, the temperature of the reaction solution was maintained at 55°C, and bromopropylmagnesium was obtained. At this time, the conversion rate of 1-bromopropane to bromopropylmagnesium was analyzed by gas chromatography and found to be 88%.

To a dried 5-L glass three-necked flask, 2 L of tetrahydrofuran (water content 10 ppm) and 24 g of methyl ethyl ketone were added, then a solution in which 50 g of bromopropylmagnesium was dissolved in 1 L of tetrahydrofuran (water content 10 ppm) was added dropwise using a dropping tube with agitation over 1 hour under an argon atmosphere, and 2-ethyl-2-pentanol was obtained. At this time, formation of 2-ethyl-2-pentanol was confirmed by $^{1}$H-NMR. In addition,

the yield of 2-ethyl-2-pentanol was confirmed by gas chromatography and found to be 83%.

[Synthesis Example 2]

To a dried 10-L glass four-necked flask, 5 L of tetrahydrofuran (water content 10 ppm) and 140 g of fine powder magnesium were added, and then the mixture was agitated at room temperature for 30 minutes under a nitrogen stream. A solution in which 1,3-dibromopropane and dichlorodimethylsilane, each component in a concentration of 2.6 mol/L, was dissolved in 1 L of tetrahydrofuran (water content 10 ppm) was added dropwise using a dropping tube at a rate of about 3 mL/min with agitation. When the temperature of the reaction solution increased by 2°C after starting the dropwise addition of the solution, the flask was cooled in a water bath set at 5°C, the temperature of the reaction solution was adjusted, and thus the temperature was maintained at about 35°C during the dropwise addition. After the dropwise addition of the solution is complete, the water bath was heated to 40°C, and the reaction solution was aged for 1 hour. Then, 530 g of triethylamine was added dropwise using a dropping tube with agitation, then the flask was cooled in an ice bath, and the reaction solution was cooled to 4°C. Then, 250 g of ethanol was added dropwise using a dropping tube with agitation over 15 minutes, then the reaction solution was filtered to remove by-product salts, and the filtrate was collected. Formation of 1,3-bis(dimethylethoxysilyl)propane was confirmed by $^1$H-NMR and $^{29}$Si-NMR. In addition, the conversion rate of 1,3-dibromopropane to 1,3-bis(dimethylethoxysilyl)propane was analyzed by an internal standard method (internal standard substance: toluene) and found to be 96%.

[Ultraviolet Irradiation Device]

A device in which two ultraviolet ray sources 11 were used in combination in the ultraviolet irradiation device 10 (see FIG. 1) and the flow tube 22 (see FIG. 2) was provided instead of the flow tube 12 was used. That is, a device in which the flow tube 22 was wound in a spiral manner around the two ultraviolet ray sources 11 was used.

Here, as the ultraviolet ray source 11, a U-tube type low-pressure mercury lamp UVC-35HU (available from Kyokko Denki Co., Ltd.) with a tube length of 280 mm and which applies ultraviolet ray with a wavelength of 254 nm was used. At this time, a fan was installed below the ultraviolet ray sources 11, and the ultraviolet ray sources 11 were forcibly air-cooled during use.

In addition, as the flow tube 22, a quartz coil with a wall thickness of 1 mm, a diameter of 10 mm, a coil diameter of 100 mm, a total length of about 10 m, and a winding number of about 30 was used. At this time, the two ultraviolet ray sources 11 were brought into contact with the flow tube 22.

[Example 1-1]

2-Ethyl-2-pentanol obtained in Synthesis Example 1 was purified as follows.

(Sample Preparation Step)

2-Ethyl-2-pentanol was dissolved in diisopropyl ether to prepare a 5 mass% solution. The lead element content in 2-ethyl-2-pentanol before purification was measured using the solution and found to be 190 ppb.

(Ultraviolet Irradiation Step)

The solution was irradiated with ultraviolet ray using the ultraviolet irradiation device. At this time, 1 L of the solution was made to flow through the flow tube 22 once at a flow rate of 60 mL/min using a tube pump and irradiated with ultraviolet ray with a wavelength of 254 nm at an illuminance of 5 mW/cm$^2$.

(Lead Component Removal Step)

The solution irradiated with ultraviolet ray was passed through a column packed with 500 mg of an iminodiacetic acid-type chelating resin, and lead ions were removed. The lead element content in 2-ethyl-2-pentanol after purification was measured using the solution passed through the column and found to be 8 ppb. In addition, the lead element removal rate was calculated by the formula:

```
100 × {1 - (lead element content after purification)/(lead

   element content before purification)}
```

The removal rate was 96%.

[Lead Element Content in Organic Compound]

In a Teflon (trade name) vessel, 1 mL of the solution was placed, then the solution was heated on a hotplate, and the organic solvents were evaporated. Then, 1 mL of ultrapure water, 3 mL of a 60 mass% nitric acid aqueous solution, and 2 mL of a 50 mass% hydrofluoric acid were added dropwise to the vessel, the mixture was heated to wet-decompose the organic compound, and then the organic compound was further heated to dryness. Wet decomposition and heating to dryness were repeated until the organic compound was completely decomposed. Then, the residue was collected with 0.2 mL of a 60 mass% nitric acid aqueous solution. A liquid of the residue made to 20 mL was used as a measurement sample.
The lead element content in the measurement sample was quantified using an Agilent 7900 ICP-MS (available from Agilent Technologies), and then the lead element content in the organic compound was determined.

[Example 1-2]

2-Ethyl-2-pentanol was purified in the same manner as in Example 1-1 except that the flow rate of 1 L of the solution flowing through the flow tube 22 was changed to 60 mL/min. At this time, the lead element content in 2-ethyl-2-pentanol after purification was 6 ppb, and the lead element removal rate was 97%.

[Example 1-3]

2-Ethyl-2-pentanol was purified in the same manner as in Example 1-1 except that instead of passing the solution irradiated with ultraviolet ray through the column, an operation of adding 3 mL of the solution irradiated with ultraviolet ray, agitating well, and then removing the aqueous phase was performed three times to remove lead salts. At this time, the lead element content in 2-ethyl-2-pentanol after purification was 4 ppb, and the lead element removal rate was 98%.

[Example 1-4]

2-Ethyl-2-pentanol was purified in the same manner as in Example 1-1 except that the flow rate of 1 L of the solution flowing through the flow tube 22 was changed to 20 mL/min. At this time, the lead element content in 2-ethyl-2-pentanol after purification was 1 ppb, and the lead element removal rate was 99%.

[Example 1-5]

2-Ethyl-2-pentanol was dissolved in tetrahydrofuran, and a 5 mass% solution was prepared. The lead element content in 2-ethyl-2-pentanol before purification was measured using the solution and found to be 210 ppb.
2-Ethyl-2-pentanol was purified in the same manner as in Example 1-2 except that the resulting solution was used. At this time, the lead element content in 2-ethyl-2-pentanol after purification was 11 ppb, and the lead element removal rate was 95%.

[Example 1-6]

2-Ethyl-2-pentanol was dissolved in n-hexane, and a 5 mass% solution was prepared. The lead element content in 2-ethyl-2-pentanol before purification was measured using the solution and found to be 203 ppb.
2-Ethyl-2-pentanol was purified in the same manner as in Example 1-2 except that the resulting solution was used. At this time, the lead element content in 2-ethyl-2-pentanol after purification was 13 ppb, and the lead element removal rate was 94%.

[Comparative Example 1]

2-Ethyl-2-pentanol was purified in the same manner as in Example 1-1 except that the solution irradiated with ultraviolet ray was not passed through the column. At this time, the lead element content in 2-ethyl-2-pentanol after purification was 189 ppb, and the lead element removal rate was 0.5%.
The evaluation results of the lead element removal rates in Examples 1-1 to 1-6 and Comparative Example 1 are shown in Table 1.

[Table 1]

| | Organic solvent | Flow rate | Lead component removal | Lead element content [ppb] | | Lead element removal rate [%] |
|---|---|---|---|---|---|---|
| | | | | Before purification | After purification | |
| Example 1-1 | Diisopropyl ether | 60 | Chelating resin | 190 | 8 | 96 |
| Example 1-2 | Diisopropyl ether | 40 | Chelating resin | 190 | 6 | 97 |
| Example 1-3 | Diisopropyl ether | 40 | Water washing | 190 | 4 | 98 |
| Example 1-4 | Diisopropyl ether | 20 | Chelating resin | 190 | 1 | 99 |
| Example 1-5 | Tetrahydrofuran | 40 | Chelating resin | 210 | 11 | 95 |
| Example 1-6 | n-Hexane | 40 | Chelating resin | 203 | 13 | 94 |
| Comparative Example 1 | Diisopropyl ether | 40 | - | 190 | 189 | 0.5 |

Table 1 shows high removal rates of the lead component in Examples 1-1 to 1-6, in which the lead component removal step was performed after the ultraviolet irradiation step without undergoing a post-treatment step, and that the method can efficiently purify 2-ethyl-2-pentanol.

In contrast to this, the lead component removal step was not performed in Comparative Example 1, and thus the lead component was not substantially removed.

[Example 2-1]

1,3-Bis(dimethylethoxysilyl)propane was purified in the same manner as in Example 1-2 except that 1,3-bis(dimethylethoxysilyl)propane obtained in Synthesis Example 2 was used instead of 2-ethyl-2-pentanol. At this time, the lead element content in 2-ethyl-2-pentanol was 98 ppb before purification and 3 ppb after purification, and the lead element removal rate was 97%.

[Example 2-2]

1,3-Bis(dimethylethoxysilyl)propane was purified in the same manner as in Example 1-3 except that 1,3-bis(dimethylethoxysilyl)propane was used instead of 2-ethyl-2-pentanol. At this time, the lead element content in 2-ethyl-2-pentanol was 98 ppb before purification and 4 ppb after purification, and the lead element removal rate was 96%.

[Example 2-3]

1,3-Bis(dimethylethoxysilyl)propane was purified in the same manner as in Example 1-5 except that 1,3-bis(dimethylethoxysilyl)propane was used instead of 2-ethyl-2-pentanol. At this time, the lead element content in 2-ethyl-2-pentanol was 91 ppb before purification and 11 ppb after purification, and the lead element removal rate was 88%.

[Example 2-4]

1,3-Bis(dimethylethoxysilyl)propane was purified in the same manner as in Example 1-6 except that 1,3-bis(dimethylethoxysilyl)propane was used instead of 2-ethyl-2-pentanol. At this time, the lead element content in 2-ethyl-2-pentanol was 103 ppb before purification and 9 ppb after purification, and the lead element removal rate was 91%.

[Comparative Example 2]

1,3-Bis(dimethylethoxysilyl)propane was purified in the same manner as in Comparative Example 1 except that 1,3-bis(dimethylethoxysilyl)propane was used instead of 2-ethyl-2-pentanol. At this time, the lead element content in 2-ethyl-2-pentanol was 98 ppb before purification and 96 ppb after purification, and the lead element removal rate was 2%.

The evaluation results of the lead element removal rates in Examples 2-1 to 2-4 and Comparative Example 2 are shown in Table 2.

[Table 2]

| | Organic solvent | Flow rate | Lead component removal | Lead element content [ppb] | | Lead element removal rate [%] |
|---|---|---|---|---|---|---|
| | | | | Before purification | After purification | |
| Example 2-1 | Diisopropyl ether | 40 | Chelating resin | 98 | 3 | 97 |
| Example 2-2 | Diisopropyl ether | 40 | Water washing | 98 | 4 | 96 |
| Example 2-3 | Tetrahydrofuran | 40 | Chelating resin | 91 | 11 | 98 |
| Example 2-4 | n-Hexane | 40 | Chelating resin | 103 | 9 | 91 |
| Comparative Example 2 | Diisopropyl ether | 40 | - | 98 | 96 | 2 |

Table 2 shows high removal rates of the lead component in Examples 2-1 to 2-4, in which the lead component removal step was performed after the ultraviolet irradiation step without undergoing a post-treatment step, and that the method can efficiently purify 1,3-bis(dimethylethoxysilyl)propane.

In contrast to this, the lead component removal step was not performed in Comparative Example 2, and thus the lead component was not substantially removed.

EXPLANATION OF REFERENCE NUMERALS

10 Ultraviolet irradiation device

11 Ultraviolet ray source

12, 22 Flow tube

**Claims**

1. An ultraviolet irradiation device comprising a flow tube through which a substance to be irradiated with ultraviolet ray flows, the flow tube being wound in a spiral manner around a bar-shaped ultraviolet ray source.

2. The ultraviolet irradiation device according to claim 1, wherein the ultraviolet irradiation device is used for purifying an organic compound containing an organic lead compound as an impurity.

3. A method for purifying an organic compound containing an organic lead compound as an impurity, the method comprising:

   a step of irradiating the organic compound with ultraviolet ray using the ultraviolet irradiation device according to claim 2; and

a step of removing a lead component that is generated as the result of the irradiation of the organic lead compound with the ultraviolet ray from the organic compound.

# FIG. 1

# FIG. 2

22

<table>
<tr><td colspan="2">**INTERNATIONAL SEARCH REPORT**</td><td>International application No.<br>**PCT/JP2022/018147**</td></tr>
</table>

**A. CLASSIFICATION OF SUBJECT MATTER**

*C07C 29/74*(2006.01)i; *C07C 31/125*(2006.01)i; *A62D 3/176*(2007.01)i; *A62D 101/24*(2007.01)n; *B01J 19/12*(2006.01)i; *B01J 19/24*(2006.01)i

FI: B01J19/12 C; C07C29/74; C07C31/125; B01J19/24 Z; A62D3/176; A62D101:24

According to International Patent Classification (IPC) or to both national classification and IPC

**B. FIELDS SEARCHED**

Minimum documentation searched (classification system followed by classification symbols)

C07B31/00-61/00; C07B63/00-63/04; C07C1/00-409/44; A62D3/176; B01J10/00-12/02; B01J14/00-19/32

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2005-013839 A (ZEOTEKKU KENKYUSHO KABUSHIKI KAISHA) 20 January 2005 (2005-01-20)<br>   claims, paragraphs [0017]-[0035], fig. 1 | 1 |
| Y | | 2-3 |
| X | JP 7-51401 A (TOSHIBA CORPORATION) 28 February 1995 (1995-02-28)<br>   paragraph [0039], fig. 1 | 1 |
| Y | | 2-3 |
| X | JP 2002-166270 A (WATANABE SHOKO KABUSHIKI KAISHA) 11 June 2002 (2002-06-11)<br>   claims, paragraphs [0014]-[0022], fig. 1 | 1 |
| Y | | 2-3 |
| Y | JP 2003-128630 A (TOKUYAMA CORPORATION) 08 May 2003 (2003-05-08)<br>   paragraph [0003] | 2-3 |

☑ Further documents are listed in the continuation of Box C.      ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **04 July 2022** | **12 July 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

<div style="text-align: center;">

**INTERNATIONAL SEARCH REPORT**

</div>

| International application No. |
| --- |
| **PCT/JP2022/018147** |

| C. | DOCUMENTS CONSIDERED TO BE RELEVANT | | |
| --- | --- | --- | --- |
| Category* | Citation of document, with indication, where appropriate, of the relevant passages | | Relevant to claim No. |
| Y | WO 2003/008339 A1 (THE ASSOCIATED OCTEL COMPANY, LIMITED) 30 January 2003 (2003-01-30)<br>    column 2, lines 5-16 | | 2-3 |
| Y | EP 0714712 A1 (DEGUSSA AKTIENGESELLSCHAFT) 28 October 1995 (1995-10-28)<br>    page 6, lines 35-49, table 3 | | 2-3 |
| Y | JP 2003-506164 A (HONEYWELL INTERNATIONAL INCORPORATED) 18 February 2003 (2003-02-18)<br>    paragraphs [0009]-[0019] | | 2-3 |

**INTERNATIONAL SEARCH REPORT**
Information on patent family members

International application No.

**PCT/JP2022/018147**

| Patent document cited in search report | | | Publication date (day/month/year) | Patent family member(s) | | | Publication date (day/month/year) |
|---|---|---|---|---|---|---|---|
| JP | 2005-013839 | A | 20 January 2005 | (Family: none) | | | |
| JP | 7-51401 | A | 28 February 1995 | (Family: none) | | | |
| JP | 2002-166270 | A | 11 June 2002 | (Family: none) | | | |
| JP | 2003-128630 | A | 08 May 2003 | (Family: none) | | | |
| WO | 2003/008339 | A1 | 30 January 2003 | EP | 1406842 | A1 | |
| EP | 0714712 | A1 | 28 October 1995 | (Family: none) | | | |
| JP | 2003-506164 | A | 18 February 2003 | US | 6498281 | B1 | |
| | | | | column 2, line 25 to column 3, line 34 | | | |
| | | | | WO | 2001/010504 | A2 | |
| | | | | CN | 1387453 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003128630 A